# EUROPÄISCHE PATENTSCHRIFT

(11) **EP 3 245 603 B1**
(45) Veröffentlichungstag und Bekanntmachung des Hinweises auf die Patenterteilung: **06.06.2018**
(21) Anmeldenummer: 16700944.8
(22) Anmeldetag: 11.01.2016
(51) Int. Cl.: G06F 19/00, A61B 5/00, B60K 28/06, G08B 25/01, A61B 5/18

(54) **VERFAHREN ZUM BEREITSTELLEN EINER MEDIZINISCHEN INFORMATION, SYSTEM UND KRAFTFAHRZEUG**
METHOD FOR PROVIDING A MEDICAL INFORMATION, SYSTEM AND MOTOR VEHICLE
PROCÉDÉ POUR FOURNIR DES INFORMATIONS MÉDICALES, SYSTÈME ET VÉHICULE À MOTEUR ASSOCIÉS

(30) Priorität: 13.01.2015 DE 102015000401
(43) Veröffentlichungstag der Anmeldung: 22.11.2017
(73) Patentinhaber: Audi AG, 85045 Ingolstadt (DE)
(72) Erfinder: NÖTH, Elmar, 97724 Burglauer (DE)
(86) Internationale Anmeldenummer: PCT/EP2016/000033
(87) Internationale Veröffentlichungsnummer: WO 2016/113126

(56) Entgegenhaltungen:
- WO-A1-02/17786
- WO-A1-02/096694
- WO-A1-2014/015990
- US-A1- 2010 234 692

## Beschreibung

Die Erfindung betrifft ein Verfahren zum Bereitstellen einer medizinischen Information eines Fahrzeuginsassen eines Kraftfahrzeugs mittels eines medizinischen Geräts des Fahrzeuginsassen, bei welchem ein die medizinische Information aufweisendes Signal an eine im Kraftfahrzeug angeordnete Einrichtung drahtlos übertragen wird. Die Erfindung betrifft außerdem ein System, wie auch ein Kraftfahrzeug.

Verfahren zum Bereitstellen einer medizinischen Information eines Fahrzeuginsassen eines Kraftfahrzeugs mittels eines medizinischen Geräts des Fahrzeuginsassen sind aus dem Stand der Technik bekannt. So ist in der DE 10 2011 016 776 A1 ein Verfahren beschrieben, bei welchem mittels eines medizinischen Geräts Gesundheitszustandsdaten eines Fahrzeuginsassen ermittelt und mittels einer Datenübertragung von dem medizinischen Gerät an das Kraftfahrzeug übertragen werden.

Die Dokumente US 2010/234692 A1 und WO 2014/015990 A1 offenbaren ein Verfahren zum Bereitstellen einer medizinischen Information eines medizinischen Geräts eines Fahrzeuginsassen, bei welchem ein die medizinische Information aufweisendes Signal an eine im Kraftfahrzeug angeordnete Einrichtung drahtlos übertragen wird. Des Weiteren wird die medizinische Information von der Einrichtung an eine kraftfahrzeugexterne Vorrichtung drahtlos übermittelt.

Es ist Aufgabe der Erfindung, ein Verfahren, ein System sowie ein Kraftfahrzeug bereitzustellen, mit welchem beziehungsweise bei welchem eine medizinische Information eines Fahrzeuginsassen eines Kraftfahrzeugs mittels eines medizinischen Geräts des Fahrzeuginsassen tiefergehender und individueller genutzt werden kann.

Diese Aufgabe wird erfindungsgemäß durch ein Verfahren, durch ein System sowie durch ein Kraftfahrzeug mit den Merkmalen gemäß den jeweiligen unabhängigen Ansprüchen gelöst.

Bei einem erfindungsgemäßen Verfahren wird eine medizinische Information eines Fahrzeuginsassen eines Kraftfahrzeugs mittels eines medizinischen Geräts des Fahrzeuginsassen bereitgestellt. Bei dem Verfahren wird ein die medizinische Information aufweisendes Signal an eine im Kraftfahrzeug angeordnete Einrichtung drahtlos übertragen. Ein wesentlicher Gedanke der Erfindung ist, dass die medizinische Information von der Einrichtung, insbesondere mittels einer Kommunikationseinheit der Einrichtung, an eine kraftfahrzeugexterne Vorrichtung drahtlos übermittelt wird.

Durch das erfindungsgemäße Verfahren wird es möglich, die medizinische Information des Fahrzeuginsassen des Kraftfahrzeugs mittels des medizinischen Geräts des Fahrzeuginsassen umfangreich und tiefergehend zu nutzen. So kann die medizinische Information in der kraftfahrzeugexternen Vorrichtung präzise und tiefgreifend analysiert werden. Abhängig von einem Ergebnis der Analyse beziehungsweise Auswertung kann dann beispielsweise eine individuelle und fachlich fundierte Konsequenz eingeleitet werden. Die Sicherheit des Kraftfahrzeugs kann dadurch erhöht werden.

Die medizinische Information ist insbesondere ein Gesundheitszustand des Fahrzeuginsassen und/oder ein Betriebszustand des medizinischen Geräts. Das medizinische Gerät ist insbesondere fest mit dem Fahrzeuginsassen verbunden. Das medizinische Gerät kann also beispielsweise als eine Insulinpumpe und/oder ein Blutzuckermessgerät und/oder ein Hörgerät und/oder ein Herzschrittmacher und/oder eine Prothese und/oder ein Dialysegerät ausgebildet sein. Das medizinische Gerät ist vorzugsweise ein aktives Gerät mit Sensoren und Parametern für eine Feinabstimmung. Das medizinische Gerät ist insbesondere nicht an dem Kraftfahrzeug angeordnet und ist somit insbesondere kein Sensor des Kraftfahrzeugs. Somit ist das medizinische Gerät nur in einem Innenraum des Kraftfahrzeugs, falls sich der Fahrzeuginsasse in dem Innenraum des Kraftfahrzeugs befindet. So ist es insbesondere vorgesehen, dass die medizinische Information über den Gesundheitszustand des Fahrzeuginsassen und/oder den Betriebszustand des medizinischen Geräts an die kraftfahrzeugexterne Vorrichtung, also beispielsweise eine Datenbank, welche sich außerhalb des Kraftfahrzeugs befindet, übermittelt wird. Daran anschließend kann die übermittelte medizinische Information automatisch und/oder durch einen menschlichen Operateur ausgewertet werden und Maßnahmen als Konsequenz eingeleitet werden. Die Maßnahmen können beispielsweise eine Übermittlung von Parametern an die Einrichtung und weiter an das medizinische Gerät sein, um den Betriebszustand des medizinischen Geräts an den Gesundheitszustand des Fahrzeuginsassen anzupassen. Weiterhin kann die medizinische Information durch die kraftfahrzeugexterne Vorrichtung so bereitgestellt werden, dass ein Rettungsdienst, welcher zu dem Kraftfahrzeug und/oder dem Fahrzeuginsassen gerufen wird, schon während der Anfahrt zu dem Kraftfahrzeug auf die medizinische Information zugreifen kann und dementsprechend frühzeitig Vorbereitung treffen kann. Hierdurch kann bei einem Notfall wertvolle Zeit gespart werden. So kann es beispielsweise auch sein, dass ein Arzt auf Basis der medizinischen Information von der Ferne aus Anweisungen an eine an dem Ort des Kraftfahrzeugs anwesende, medizinisch ungeschulte Person erteilt. Dem Fahrzeuginsassen kann somit ebenfalls frühzeitig Unterstützung bereitgestellt werden.

Weiterhin vorteilhaft ist, dass der Gesundheitszustand insbesondere ohne eine explizite Handlung des Fahrzeuginsassen festgestellt werden kann. Das heißt, dass der Fahrzeuginsasse das Übertragen der medizinischen Information vorzugsweise gar nicht bemerkt. Somit kann sich der Fahrzeuginsasse insbesondere auf das Führen des Kraftfahrzeugs konzentrieren. Der Fahrzeuginsasse ist jedoch aufgrund der fahrzeugexternen Vorrichtung insbesondere jederzeit mit einer geeigneten Stelle verbunden, um die medizinische Information auszuwerten und/oder zu überwachen und/oder zu der geeigneten Stelle zu übertragen, und gegebenenfalls darauf zu reagieren.

Die drahtlose Übertragung des Signals mit der medizinischen Information erfolgt im Bereich von Schallwellen, also akustisch, beispielsweise mit Ultraschallsendern und -empfängern. Ergänzend oder alternativ kann es vorgesehen sein, dass die medizinische Information über Sprachsignale, also eine Sprachausgabe und eine Spracherkennung ausgetauscht wird. So kann das medizinische Gerät beispielsweise dazu ausgelegt sein, den Gesundheitszustand und/oder den Betriebszustand mittels Sprache beziehungsweise Wörtern auszugeben. Die Sprache wiederum kann dann beispielsweise von der Einrichtung erkannt werden und an die kraftfahrzeugexterne Vorrichtung übermittelt werden. Die drahtlose Übermittlung der medizinischen Information von der Einrichtung an die kraftfahrzeugexterne Vorrichtung kann beispielsweise mit einem Mobilfunkstandard erfolgen. Für die drahtlose Übermittlung der medizinischen Information an die kraftfahrzeugexterne Vorrichtung kann aber beispielsweise auch eine bereits in dem Kraftfahrzeug vorhandene, von der Einrichtung separate Kommunikationseinrichtung des Kraftfahrzeugs genutzt werden.

Insbesondere ist vorgesehen, dass das medizinische Gerät fest mit dem Körper des Fahrzeuginsassen verbunden wird und/oder als in den Körper des Fahrzeuginsassen implantiertes medizinisches Gerät ausgebildet wird. Das bedeutet, dass das medizinische Gerät nicht an dem Kraftfahrzeug angeordnet ist und somit nicht als Sensor des Kraftfahrzeugs ausgebildet ist. Ein Verlassen des Innenraums des Kraftfahrzeugs durch den Fahrzeuginsassen führt insbesondere dazu, dass das medizinische Gerät ebenfalls nicht mehr in dem Innenraum vorhanden ist. Das medizinische Gerät kann beispielsweise als implantierter Defibrillator und/oder Herzschrittmacher und/oder Insulinpumpe und/oder Blutzuckermessgerät und/oder Hörgerät und/oder Prothese und/oder Dialysegerät ausgelegt sein. Das medizinische Gerät weist insbesondere eine Schnittstelle auf, welche mit einer Schnittstelle der Einrichtung kompatibel ist, um das Signal mit der medizinischen Information drahtlos zu übertragen. Vorteilhaft ist, dass das fest mit dem Körper des Insassen verbundene medizinische Gerät präzisere und weitergehende medizinische Informationen über den Gesundheitszustand des Fahrzeuginsassen bereitstellen kann, als dies beispielsweise mit Sensoren des Kraftfahrzeugs ermöglicht wird.

Weiterhin ist es insbesondere vorgesehen, dass die kraftfahrzeugexterne Vorrichtung als eine Datenbank, insbesondere einer medizinischen Versorgungseinrichtung und/oder eines Herstellers des medizinischen Geräts, bereitgestellt wird. Die medizinische Versorgungseinrichtung kann beispielsweise als ein Krankenhaus und/oder eine Arztpraxis ausgebildet sein. Weiterhin kann es vorgesehen sein, dass die Datenbank durch einen Hersteller des Kraftfahrzeugs bereitgestellt wird. Die Datenbank kann beispielsweise mit einem relationalen Datenbankmodell oder einem nicht-relationalen Datenbankmodell abgebildet werden. Vorzugsweise kann es auch sein, dass die Datenbank an weitere Datenbanken übermittelt wird, um die medizinische Information an mehreren Orten gleichzeitig bereitstellen zu können. So kann die Datenbank beispielsweise auch in einem Rettungswagen vorliegen. Es kann auch vorgesehen sein, dass die medizinische Information in der Datenbank mit historischer medizinischer Information des Fahrzeuginsassen ergänzt wird. Die historische medizinische Information kann beispielsweise zu einem früheren Zeitpunkt als die aktuell an die kraftfahrzeugexterne Vorrichtung übermittelte medizinische Information erfasst worden sein. Aufgrund der historischen medizinischen Information ist die Datenbank noch aussagekräftiger und Maßnahmen zur Unterstützung des Fahrzeuginsassen können effektiver eingeleitet werden.

Vorzugsweise ist es vorgesehen, dass die medizinische Information mittels der kraftfahrzeugexternen Vorrichtung ausgewertet wird, und ein Ergebnis der Auswertung von der kraftfahrzeugexternen Vorrichtung an die Einrichtung übermittelt wird, wobei zumindest ein Parameter des medizinischen Geräts abhängig von dem übermittelten Ergebnis durch Übersendung eines Anpassungssignals von der Einrichtung an das medizinische Gerät angepasst wird. Das Auswerten der medizinischen Information kann beispielsweise automatisch auf Basis von vorgegebenen Vorgehensweisen erfolgen. Ergänzend oder alternativ kann die Auswertung allerdings auch durch eine Person, beispielsweise einen Arzt, erfolgen. Abhängig von der Auswertung kann ein Ergebnis bestimmt werden und an die Einrichtung übertragen beziehungsweise übermittelt werden. Die Einrichtung kann nun abhängig von dem übermittelten Ergebnis das Anpassungssignal an das medizinische Gerät senden, um zumindest ein Parameter des medizinischen Geräts anzupassen. Es kann also beispielsweise eine Einstellung der Insulinpumpe geändert werden, so dass mehr oder weniger Insulin an den Fahrzeuginsassen abgegeben wird. Vorteilhaft ist also, dass der Parameter des medizinischen Geräts fachkundig auf Basis der in der kraftfahrzeugexternen Vorrichtung vorhandenen medizinischen Information individuell und aktuell angepasst werden kann. Aktuell bedeutet also, dass der Parameter live beziehungsweise in Echtzeit angepasst werden kann.

Weiterhin ist vorzugsweise vorgesehen, dass die medizinische Information mittels der kraftfahrzeugexternen Vorrichtung ausgewertet wird, und abhängig eines von der kraftfahrzeugexternen Vorrichtung an die Einrichtung übermittelten Ergebnisses der Auswertung eine erste Mitteilung an den Fahrzeuginsassen und/oder einen weiteren Fahrzeuginsassen des Kraftfahrzeugs im Kraftfahrzeug ausgegeben wird, und insbesondere durch die Einrichtung eine Reaktion des Fahrzeuginsassen und/oder des weiteren Fahrzeuginsassen auf die erste Mitteilung ermittelt wird. Es kann also auf Basis des übermittelten Ergebnisses der Auswertung die erste Mitteilung an den Fahrzeuginsassen und/oder den weiteren Fahrzeuginsassen erfolgen. So kann dem Fahrzeuginsassen beispielsweise in Form einer visuellen Meldung angezeigt werden, welches Ergebnis die Auswertung ergeben hat und beispielsweise welche Konsequenzen und/oder Handlungsanweisungen daraus folgen beziehungsweise welche Handlungsschritte eingeleitet werden können. Ergänzend oder alternativ kann die erste Mitteilung auch an den weiteren Fahrzeuginsassen, welcher nicht fest mit dem medizinischen Gerät verbunden ist, ausgegeben werden, um von dem weiteren Fahrzeuginsassen Hilfe beziehungsweise Unterstützung für den Fahrzeuginsassen mit dem medizinischen Gerät zu erhalten. Es kann somit auch eine konkrete Handlungsanweisung an den weiteren Fahrzeuginsassen ausgegeben werden. Durch das Ermitteln der Reaktion kann festgestellt werden, ob die zweite Mitteilung von dem Fahrzeuginsassen und/oder dem weiteren Fahrzeuginsassen korrekt aufgenommen beziehungsweise verstanden worden ist. So kann durch die Reaktion beispielsweise eine Testfrage oder eine Testreaktion abgefragt und ermittelt werden. Die Testreaktion ermöglicht das Testen eines physischen und/oder psychischen Zustands des Fahrzeuginsassen.

Vorzugsweise ist es vorgesehen, dass die erste Mitteilung visuell und/oder haptisch und/oder akustisch in dem Kraftfahrzeug ausgegeben wird. Visuell kann die erste Mitteilung beispielsweise auf einem Bildschirm des Kraftfahrzeugs ausgegeben werden. Haptisch kann die erste Mitteilung beispielsweise durch eine Vibration des Lenkrads des Kraftfahrzeugs ausgegeben werden. Akustisch wird die erste Mitteilung beispielsweise über ein Lautsprechersystem des Kraftfahrzeugs ausgegeben. Die erste Mitteilung kann beispielsweise akustisch als Warnton oder als Sprachausgabe, beispielsweise mit konkreten aktuellen Handlungshinweisen, ausgegeben werden. Vorteilhaft ist, dass der Fahrzeuginsasse und/oder der weitere Fahrzeuginsasse vielfältig und somit deutlich durch die erste Mitteilung auf das übermittelte Ergebnis hingewiesen werden kann.

In einer weiteren Ausführungsform ist es vorzugsweise vorgesehen, dass die medizinische Information mittels der kraftfahrzeugexternen Vorrichtung ausgewertet wird und abhängig von dem Ergebnis der Auswertung eine zweite Mitteilung an eine kraftfahrzeugexterne Person, insbesondere eine medizinisch geschulte Person, durch die kraftfahrzeugexterne Vorrichtung ausgegeben wird. Die kraftfahrzeugexterne Person kann beispielsweise ein Arzt und/oder ein Rettungssanitäter in einem Rettungswagen sein. Ergänzend oder alternativ kann der Arzt beispielsweise auch in einem Krankenhaus auf eine Einlieferung des Fahrzeuginsassen warten und zur Vorbereitung auf den Fahrzeuginsassen mit der zweiten Mitteilung frühzeitig, beispielsweise auf eine bevorstehende Operation, eingestellt werden. Dadurch kann eine gut vorbereitete Behandlung des Fahrzeuginsassen erfolgen.

In einer weiteren Ausführungsform ist vorzugsweise vorgesehen, dass die medizinische Information mittels der kraftfahrzeugexternen Vorrichtung ausgewertet wird, und abhängig von einem Ergebnis der Auswertung ein Eingriff in die Steuerung des Kraftfahrzeugs ausgeführt wird. So kann das Ergebnis der Auswertung beispielsweise von der kraftfahrzeugexternen Vorrichtung an die Einrichtung zurückübertragen werden und dadurch die Steuerung des Kraftfahrzeugs beeinflusst werden. Der Eingriff in die Steuerung des Kraftfahrzeugs umfasst beispielsweise ein Bremsen des Kraftfahrzeugs und/oder ein Beschleunigen des Kraftfahrzeugs und/oder ein Lenken des Kraftfahrzeugs und/oder eine von einem Navigationssystem des Kraftfahrzeugs vorgeschlagene Fahrtroute. Durch die Steuerung des Kraftfahrzeugs sind also insbesondere sämtliche Fahrzeugparameter des Kraftfahrzeugs umfasst, welche eine Bewegung des Kraftfahrzeugs mittelbar oder unmittelbar betreffen. So kann das Kraftfahrzeug beispielsweise aufgrund des Ergebnisses durch eine Notbremsung gestoppt werden. Ergänzend oder Alternativ kann dem Fahrer des Kraftfahrzeugs, insbesondere dem Fahrzeuginsassen, auch eine aktuelle Fahrtroute durch das Navigationssystem ausgegeben werden, um den Fahrzeuginsassen schnellstmöglich zu einem Ort zu navigieren, an welchem dieser bezüglich seines Gesundheitszustands bestmöglich behandelt werden kann. Durch das Auswerten der medizinischen Information mittels der kraftfahrzeugexternen Vorrichtung kann genauer in die Steuerung des Fahrzeugs eingegriffen werden, als wenn eine Auswertung der medizinischen Information ausschließlich innerhalb des Kraftfahrzeugs erfolgen würde.

Weiterhin ist es vorzugsweise vorgesehen, dass der Fahrzeuginsasse durch das Übertragen der medizinischen Information an die Einrichtung ein notwendiges Übertragen der medizinischen Information, beispielsweise zu Hause, substituieren kann. So kann es beispielsweise vorgesehen sein, dass der Fahrzeuginsasse die medizinische Information von dem medizinischen Gerät zu vorbestimmten Zeitpunkten, beispielsweise einmal täglich, abrufen soll. Das Abrufen der medizinischen Information zu dem vorbestimmten Zeitpunkt kann somit während der Nutzung des Kraftfahrzeugs erfolgen und es muss keine weitere Zeit während des Tages aufwendet werden, um die medizinische Information abzurufen beziehungsweise abzufragen. Dadurch kann der Alltag des Fahrzeuginsassen effizienter gestaltet werden.

Weiterhin betrifft die Erfindung ein System zum Bereitstellen einer medizinischen Information eines Fahrzeuginsassen eines Kraftfahrzeugs mittels eines medizinischen Geräts des Fahrzeuginsassen, welches ein Kraftfahrzeug mit einer internen Einrichtung beziehungsweise einer Einrichtung aufweist, und mit einer kraftfahrzeugexternen Vorrichtung, wobei das System derart ausgebildet ist, dass ein die medizinische Information aufweisendes Signal an die im Kraftfahrzeug angeordnete Einrichtung drahtlos übertragen wird und die medizinische Information von der Einrichtung an die kraftfahrzeugexterne Vorrichtung drahtlos übermittelt wird. Das System ist dazu ausgebildet, ein erfindungsgemäßes Verfahren oder eine vorteilhafte Ausführung davon auszuführen.

Ein erfindungsgemäßes Kraftfahrzeug umfasst eine internen Einrichtung, welche zur drahtlosen Kommunikation mit einem medizinischen Gerät eines Fahrzeuginsassen und zur drahtlosen Kommunikation mit einer kraftfahrzeugexternen Vorrichtung zur drahtlosen Übertragung eines den Fahrzeuginsassen betreffende medizinische Information aufweisenden Signals mittels des medizinischen Geräts ausgebildet ist.

Die mit Bezug auf das erfindungsgemäße Verfahren vorgestellten bevorzugten Ausführungsformen und deren Vorteile gelten entsprechend für das erfindungsgemäße System sowie für das erfindungsgemäße Kraftfahrzeug.

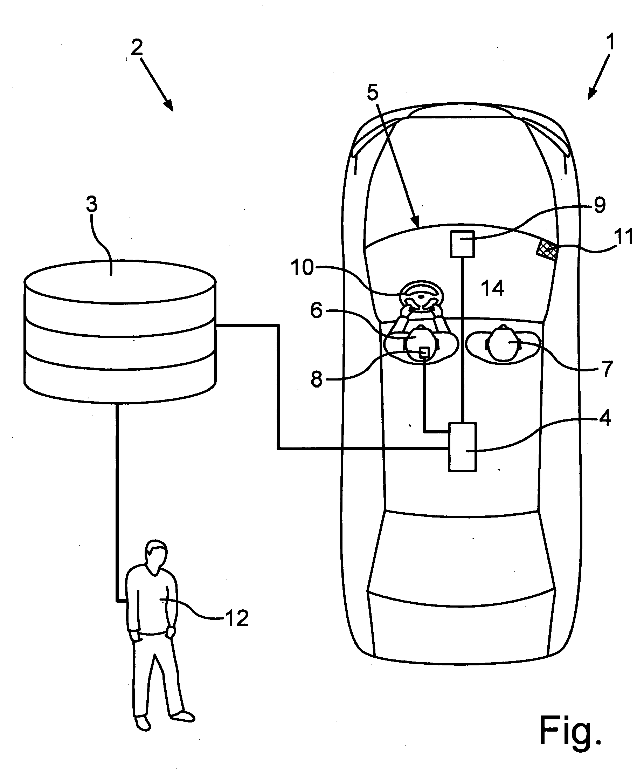

Weitere Merkmale der Erfindung ergeben sich aus den Ansprüchen, der Figur und der Figurenbeschreibung. Alle vorstehend in der Beschreibung genannten Merkmale und Merkmalskombinationen sowie die nachfolgend in der Figurenbeschreibung genannten und/oder in der Figur alleine gezeigten Merkmale und Merkmalskombinationen sind nicht nur in der jeweils angegebenen Kombination, sondern auch in anderen Kombinationen oder aber in Alleinstellung verwendbar, soweit sie den Rahmen der Erfindung nicht verlassen. Es sind somit auch beanspruchte Ausführungen von der Erfindung als umfasst und offenbart anzusehen, die in der Figur nicht explizit gezeigt und erläutert sind, jedoch durch separierte Merkmalskombinationen aus den erläuterten Ausführungen hervorgehen und erzeugbar sind. Es sind auch beanspruchte Ausführungen und Merkmalskombinationen als offenbart anzusehen, die somit nicht alle Merkmale eines ursprünglich formulierten unabhängigen Anspruchs aufweisen.

Die Erfindung wird nun anhand eines bevorzugten Ausführungsbeispiels sowie unter Bezugnahme auf die beigefügte Zeichnung näher erläutert.

Dabei zeigt die Fig. eine schematische Darstellung eines Ausführungsbeispiels eines erfindungsgemäßen Kraftfahrzeugs mit einer internen Einrichtung, und einer kraftfahrzeugexternen Vorrichtung.

Die Fig. zeigt eine schematische Darstellung eines Kraftfahrzeugs 1 und eines Systems 2. Das System 2 umfasst das Kraftfahrzeug 1 und eine kraftfahrzeugexterne Vorrichtung 3. Die kraftfahrzeugexterne Vorrichtung 3 ist vorzugsweise als Datenbank ausgebildet und beispielsweise in einem Krankenhaus und/oder in einer Arztpraxis und/oder bei einem Hersteller des Kraftfahrzeugs 1 vorgehalten.

Das Kraftfahrzeug 1 umfasst eine Einrichtung 4. In einem Innenraum 5 des Kraftfahrzeugs 1 befindet sich ein Fahrzeuginsasse 6 und ein weiterer Fahrzeuginsasse 7. Gemäß dem Ausführungsbeispiel ist der Fahrzeuginsasse 6 als der Fahrer des Kraftfahrzeugs 1 vorgesehen und der weitere Fahrzeuginsasse 7 als der Beifahrer des Kraftfahrzeugs 1 vorgesehen. Es kann jedoch auch sein, dass der weitere Fahrzeuginsasse 7 der Fahrer des Kraftfahrzeugs 1 ist oder, dass weder der Fahrzeuginsasse 6 noch der weitere Fahrzeuginsasse 7 Fahrer des Kraftfahrzeugs 1 sind.

An beziehungsweise in dem Fahrzeuginsassen 6 ist ein medizinisches Gerät 8 angeordnet. Das medizinische Gerät 8 kann beispielsweise als eine Insulinpumpe und/oder ein Blutzuckermessgerät und/oder ein Hörgerät und/oder ein Herzschrittmacher und/oder eine Prothese und/oder ein Dialysegerät und/oder ein implantierter Defibrillator ausgebildet sein. Das medizinische Gerät 8 und die Einrichtung 4 verfügen jeweils über Schnittstellen und sind dazu ausgelegt, miteinander medizinische Informationen auszutauschen. Es kann also medizinische Information drahtlos von dem medizinischen Gerät 8 an die Einrichtung 4 übertragen werden. Die drahtlose Übertragung erfolgt mittels akustischer Übertragung, beispielsweise mittels Ultraschall. Die medizinische Information wird insbesondere durch einen Gesundheitszustand des Fahrzeuginsassen 6 und/oder einem Betriebszustand des medizinischen Geräts 8 charakterisiert.

Die Einrichtung 4 ist gemäß dem Ausführungsbeispiel zentral an dem Kraftfahrzeug 1 angeordnet, jedoch ist die Anordnung der Einrichtung 4 vielfältig an dem Kraftfahrzeug 1 möglich, vorzugsweise allerdings so, dass eine Kommunikation beziehungsweise ein Übertragen der medizinischen Information von dem medizinischen Gerät 8 zu der Einrichtung 4 drahtlos beziehungsweise kabellos möglich ist.

Es ist nun vorgesehen, dass die medizinische Information von der Einrichtung 4 drahtlos an die kraftfahrzeugexterne Vorrichtung 3 übermittelt wird. Dies kann beispielsweise manuell ausgelöst durch den Fahrzeuginsassen 6 erfolgen und/oder automatisch, beispielsweise in regelmäßigen Abständen, durch die Einrichtung 4 durchgeführt werden. Die medizinische Information kann nun mittels der kraftfahrzeugexternen Vorrichtung 3 ausgewertet werden. Als Folge der Auswertung wird ein Ergebnis bereitgestellt. Die Auswertung kann beispielsweise automatisch durch vorgegebene Ablaufschemen und/oder durch eine Person erfolgen. Die Person kann beispielsweise ein Arzt und/oder eine für den konkreten Bedarf des Fahrzeuginsassen 6 anderweitig medizinisch geschulte Person sein.

Das Ergebnis kann nun an die Einrichtung 4 übertragen werden, um beispielsweise einen Parameter des medizinischen Geräts 8 abhängig von dem übermittelten Ergebnis anzupassen. Hierzu wird insbesondere ein Anpassungssignal von der Einrichtung 4 an das medizinische Gerät 8 übersendet. Es wird also insbesondere zuerst das Ergebnis von der kraftfahrzeugexternen Vorrichtung 3 an die Einrichtung 4 übertragen und anschließend wird das Anpassungssignal mit zumindest einem Parameter des medizinischen Geräts 8 von der Einrichtung 4 an das medizinische Gerät übersendet. Der Parameter kann von dem Ergebnis umfasst werden oder aber bereits in der Einrichtung 4 vorgehalten werden.

Das Ergebnis der Auswertung kann aber auch von der kraftfahrzeugexternen Vorrichtung 3 an die Einrichtung 4 übermittelt werden, um durch die Einrichtung 4 eine erste Mitteilung, beispielsweise auf einer Anzeigeeinheit 9 des Kraftfahrzeugs 1 auszugeben. Die erste Mitteilung ist beispielsweise ein Warnhinweis und/oder eine Behandlungsanleitung. Die erste Mitteilung kann also beispielsweise visuell auf der Anzeigeeinheit 9 ausgegeben werden. Ergänzend oder alternativ kann die erste Meldung beispielsweise haptisch an einem Lenkrad 10 des Kraftfahrzeugs 1 ausgegeben werden. Die haptische Ausgabe kann beispielsweise durch eine Vibration beziehungsweise ein Rütteln des Lenkrads 10 erfolgen. Weiterhin kann die erste Meldung zusätzlich oder anstatt akustisch durch einen Lautsprecher 11 des Kraftfahrzeugs 1 ausgegeben werden. Somit kann der Fahrzeuginsasse 6 und/oder der weitere Fahrzeuginsasse 7 durch die Anzeigeeinheit 9 und/oder das Lenkrad 10 und/oder den Lautsprecher 11 auf die erste Mitteilung hingewiesen werden. Weiterhin kann beispielsweise eine Reaktion von dem Fahrzeuginsassen 6 und/oder dem weiteren Fahrzeuginsassen 7 auf die erste Mitteilung ermittelt werden. So kann beispielsweise überprüft werden, ob die erste Mitteilung registriert worden ist. Falls keine Reaktion oder eine andere Reaktion als erwartet von dem Fahrzeuginsassen 6 erfolgt, so kann dies von der Einrichtung 4 an die kraftfahrzeugexterne Vorrichtung 3 übermittelt werden und in eine nachfolgende Auswertung der medizinischen Daten einfließen. So kann also überprüft werden, ob die erste Mitteilung von dem Fahrzeuginsassen 6 und/oder dem weiteren Fahrzeuginsassen 7 registriert beziehungsweise richtig registriert wurde. Weiterhin kann dadurch ermittelt werden, ob der Fahrzeuginsasse 6 beispielsweise bei Bewusstsein ist.

Das Ergebnis der Auswertung kann ergänzend oder alternativ als eine zweite Mitteilung an eine kraftfahrzeugexterne Person 12 übermittelt werden. Die zweite Mitteilung umfasst beispielsweise detaillierte Informationen über den aktuellen Gesundheitszustand des Fahrzeuginsassen 6. Die kraftfahrzeugexterne Person 12 kann beispielsweise eine medizinisch geschulte Person, wie ein Arzt oder ein Rettungssanitäter sein. Durch die kraftfahrzeugexterne Person 12 kann beispielsweise eine Anleitung zur Behandlung des Fahrzeuginsassen 6 an den weiteren Fahrzeuginsassen 7 gegeben werden. Aufgrund der medizinischen Information kann sich die kraftfahrzeugexterne Person 12 aber auch vor einem möglicherweise bevorstehenden Rettungseinsatz vorbereiten, um die Behandlung des Fahrzeuginsassen 6 nach dem Eintreffen vor Ort möglichst zeitnah und effektiv durchzuführen.

Ergänzend oder alternativ kann das Ergebnis, welches von der kraftfahrzeugexternen Vorrichtung an die Einrichtung 4 übertragen wird, auch genutzt werden, um einen Eingriff in die Steuerung des Kraftfahrzeugs 1 auszuführen. Der Eingriff in die Steuerung des Kraftfahrzeugs 1 kann beispielsweise ein Bremsen, insbesondere eine Notbremsung, oder ein Beschleunigen und/oder ein Lenken des Kraftfahrzeugs 1 sein. Durch die Steuerung kann aber auch in vielfältiger Weise ein Steuerbefehl an eine Steuereinheit des Kraftfahrzeugs 1 erteilt werden. So kann beispielsweise auch ein Abstandsregeltempomat (ACC - Adaptive Cruise Control) des Kraftfahrzeugs 1 abhängig von dem Ergebnis angepasst werden. Ist aufgrund des Ergebnisses also bekannt, dass der Fahrzeuginsasse 6, insbesondere der Fahrer des Kraftfahrzeugs 1, in seiner Wahrnehmung der aktuellen Verkehrssituation eingeschränkt ist, so kann ein Mindestabstand des Kraftfahrzeugs 1 zu einem vorausfahrenden Kraftfahrzeug erhöht werden. So kann die medizinische Information in Form des Gesundheitszustands des Fahrzeuginsassen 6 beispielsweise durch einen niedrigen Blutzuckerspiegel charakterisiert sein. Die Auswertung dieser medizinischen Information kann dann dazu führen, dass mit Symptomen wie einer Trübung des Bewusstseins des Fahrzeuginsassen 6 gerechnet werden muss. Schließlich kann das Ergebnis, welches an die Einrichtung 4 übertragen wird, dazu führen, dass die erste Mitteilung und/oder die zweite Mitteilung ausgegeben wird und/oder der Eingriff in die Steuerung des Kraftfahrzeugs ausgeführt wird.

Weiterhin kann durch den Eingriff in die Steuerung des Kraftfahrzeugs 1 ein Anpassen des, von beispielsweise einem Navigationssystem des Kraftfahrzeugs 1, ausgegebenen Navigationswegs beziehungsweise Navigationsvorschlags des Kraftfahrzeugs 1 ausgeführt werden. Somit kann das Kraftfahrzeug 1 beispielsweise auf schnellstem Weg zu einem Krankenhaus beziehungsweise einer medizinischen Versorgungseinrichtung geleitet werden.

Weiterhin kann durch den Eingriff in die Steuerung des Kraftfahrzeugs 1 und/oder durch die Einrichtung 4 selbst ein Notruf aufgrund des Ergebnisses der Auswertung der medizinischen Information beispielsweise an eine Notrufzentrale ausgeführt werden. Hierzu kann beispielsweise auch der aktuelle Standort des Kraftfahrzeugs 1, welcher beispielsweise mittels eines GNSS-Empfängers (GNSS - Global Navigation Satellite System) des Kraftfahrzeugs 1 ermittelt worden ist, berücksichtigt werden.

Weiter kann es vorgesehen sein, dass die kraftfahrzeugexterne Vorrichtung 3 auch historische medizinische Informationen des Fahrzeuginsassen 6 umfasst. Die historische medizinische Information kann beispielsweise einen vollständigen Krankheitsverlauf des Fahrzeuginsassen 6, beispielsweise der vorhergegangenen Tage oder der vorhergegangenen Monate, bereitstellen. Die historische medizinische Information kann dann beispielsweise in der kraftfahrzeugexternen Vorrichtung 3 mit der medizinischen Information beziehungsweise der aktuellen medizinischen Information, welche durch die Einrichtung 4 an die kraftfahrzeugexterne Vorrichtung 3 übermittelt wird, fusioniert werden.

## Patentansprüche

1. Verfahren zum Bereitstellen einer medizinischen Information eines Fahrzeuginsassen (6) eines Kraftfahrzeugs (1) mittels eines medizinischen Geräts (8) des Fahrzeugsinsassen (6), bei welchem ein die medizinische Information aufweisendes Signal an eine im Kraftfahrzeug (1) angeordnete Einrichtung (4) drahtlos übertragen wird, und die medizinische Information von der Einrichtung (4) an eine kraftfahrzeugexterne Vorrichtung (3) drahtlos übermittelt wird,
**dadurch gekennzeichnet, dass**
das die medizinische Information aufweisende Signal drahtlos im Bereich von Schallwellen akustisch zur im Kraftfahrzeug (1) angeordneten Einrichtung (4) übertragen wird.

2. Verfahren nach Anspruch 1,
**dadurch gekennzeichnet, dass**
das medizinische Gerät (8) fest, und somit nicht als Sensor des Kraftfahrzeugs, mit dem Körper des Fahrzeuginsassen (6) verbunden wird und/oder als in den Körper des Fahrzeugsinsassen (6) implantiertes medizinisches Gerät (8) ausgebildet wird.

3. Verfahren nach Anspruch 1 oder 2,
**dadurch gekennzeichnet, dass**
die kraftfahrzeugexterne Vorrichtung (3) als eine Datenbank, insbesondere einer medizinischen Versorgungseinrichtung und/oder eines Herstellers des medizinischen Geräts (8), bereitgestellt wird.

4. Verfahren nach einem der vorhergehenden Ansprüche,
**dadurch gekennzeichnet, dass**
die medizinische Information mittels der kraftfahrzeugexternen Vorrichtung (3) ausgewertet wird, und abhängig eines von der kraftfahrzeugexternen Vorrichtung (3) an die Einrichtung (4) übermittelten Ergebnisses der Auswertung eine auf das übermittelte Ergebnis hinweisende Mitteilung an den Fahrzeuginsassen (6) und/oder einen weiteren Fahrzeuginsassen (7) des Kraftfahrzeugs (1) im Kraftfahrzeug (1) ausgegeben wird, und insbesondere durch die Einrichtung (4) eine Reaktion des Fahrzeuginsassen (6) und/oder des weiteren Fahrzeuginsassen (7) auf die erste Mitteilung ermittelt wird.

5. Verfahren nach Anspruch 4,
**dadurch gekennzeichnet, dass**
die erste Mitteilung visuell und/oder haptisch und/oder akustisch ausgegeben wird.

6. Verfahren nach einem der vorhergehenden Ansprüche,
**dadurch gekennzeichnet, dass**
die medizinische Information mittels der kraftfahrzeugexternen Vorrichtung (3) ausgewertet wird, und abhängig von einem Ergebnis der Auswertung eine auf den Fahrzeuginsassen vorbereitende Mitteilung an einen Arzt in einem Krankenhaus durch die kraftfahrzeugexterne Vorrichtung (3) ausgegeben wird.

7. Verfahren nach einem der vorhergehenden Ansprüche,
**dadurch gekennzeichnet, dass**
die medizinische Information mittels der kraftfahrzeugexternen Vorrichtung (3) ausgewertet wird, und abhängig von einem Ergebnis der Auswertung ein Eingriff in die Steuerung des Kraftfahrzeugs (1) ausgeführt wird.

8. Verfahren nach einem der vorhergehenden Ansprüche,
**dadurch gekennzeichnet, dass**
das die medizinische Information aufweisende Signal drahtlos im Bereich von Ultraschallwellen akustisch übertragen wird.

9. System zum Bereitstellen einer medizinischen Information eines Fahrzeuginsassen (6) eines Kraftfahrzeugs (1), wobei das System ein Kraftfahrzeug (1) gemäß Anspruch 11 umfasst, und wobei das System derart ausgebildet ist, dass die medizinische Information von der Einrichtung (4) an die kraftfahrzeugexterne Vorrichtung (3) drahtlos übermittelt wird.

10. System nach Anspruch 9,
**dadurch gekennzeichnet, dass**
die kraftfahrzeugexterne Vorrichtung (3) dazu eingerichtet ist, die medizinische Information auszuwerten und ein Ergebnis der Auswertung an die interne Einrichtung (4) des Kraftfahrzeugs zu übermitteln, wobei das medizinische Gerät (8) dazu eingerichtet ist, zumindest einen Parameter des medizinischen Geräts (8) abhängig von dem übermittelten Ergebnis durch Übersendung eines Anpassungssignals von der internen Einrichtung (4) an das medizinische Gerät (8) anzupassen.

11. Kraftfahrzeug (1) mit einer internen Einrichtung (4), welche zur drahtlosen Kommunikation eines eine den Fahrzeuginsassen betreffende medizinische Information aufweisenden Signals mittels eines medizinischen Geräts (8) eines Fahrzeuginsassen (6) und zur drahtlosen Kommunikation mit einer kraftfahrzeugexternen Vorrichtung (3) zur drahtlosen Übertragung eines die medizinische Information aufweisenden Signals ausgebildet ist, wobei das die medizinische Information aufweisende Signal drahtlos im Bereich von Schallwellen akustisch zur internen Einrichtung (4) übertragen wird.

## Claims

1. Method for providing medical information of a vehicle occupant (6) of a motor vehicle (1) by means of a medical device (8) of the vehicle occupant (6), in which a signal comprising the medical information is transmitted wirelessly to equipment (4) arranged in the motor vehicle (1) and the medical information is transmitted wirelessly from the equipment (4) to an apparatus (3) external to the motor vehicle,
**characterised in that**
the signal comprising the medical information is transmitted acoustically and wirelessly in the range of soundwaves to the equipment (4) arranged in the motor vehicle (1).

2. Method according to claim 1,
**characterised in that**
the medical device (8) is connected permanently, and thus not as a sensor of the motor vehicle, to the body of the vehicle occupant (6) and/or is designed as a medical device (8) implanted in the body of the vehicle occupant (6).

3. Method according to claim 1 or 2,
**characterised in that**
the apparatus (3) external to the motor vehicle is provided as a database, in particular of a medical care facility and/or of a manufacturer of the medical device (8).

4. Method according to any of the preceding claims,
**characterised in that**
the medical information is evaluated by means of the apparatus (3) external to the motor vehicle and, based on a finding of the evaluation transmitted to the equipment (4) from the apparatus (3) external to the motor vehicle, a message to the vehicle occupant (6) and/or an additional vehicle occupant (7) of the motor vehicle (1) is output in the motor vehicle (1) indicating the transmitted finding, and in particular, a reaction to the first message by the vehicle occupant (6) and/or the additional vehicle occupant (7) is determined by the equipment (4).

5. Method according to claim 4,
**characterised in that**
the first message is output visually and/or haptically and/or acoustically.

6. Method according to any of the preceding claims,
**characterised in that**
the medical information is evaluated by means of the apparatus (3) external to the motor vehicle and, based on a finding of the evaluation, a message to a doctor in a hospital preparing the doctor for the vehicle occupant is output by the apparatus (3) external to the motor vehicle.

7. Method according to any of the preceding claims,
**characterised in that**
the medical information is evaluated by means of the apparatus (3) external to the motor vehicle and, based on a finding of the evaluation, an intervention in the control of the motor vehicle (1) is executed.

8. Method according to any of the preceding claims,
**characterised in that**
the signal comprising the medical information is transmitted acoustically and wirelessly in the range of ultrasound waves.

9. System for providing medical information of a vehicle occupant (6) of a motor vehicle (1), wherein the system comprises a motor vehicle (1) according to claim 11, and wherein the system is designed in such a way that the medical information is transmitted wirelessly from the equipment (4) to the apparatus (3) external to the motor vehicle.

10. System according to claim 9,
**characterised in that**
the apparatus (3) external to the motor vehicle is configured to evaluate the medical information and transmit a finding of the evaluation to the internal equipment (4) of the motor vehicle, wherein the medical device (8) is configured to adapt at least one parameter of the medical device (8) based on the transmitted finding by receiving an adaptation signal from the internal equipment (4).

11. Motor vehicle (1) comprising internal equipment (4) designed to wirelessly communicate a signal comprising medical information concerning the vehicle occupant by means of a medical device (8) of a vehicle occupant (6) and to wirelessly communicate with an apparatus (3) external to the motor vehicle in order to wirelessly transmit a signal comprising the medical information, wherein the signal comprising the medical information is transmitted wirelessly in the range of soundwaves to the internal equipment (4).

## Revendications

1. Procédé pour fournir une information médicale d'un occupant de véhicule (6) d'un véhicule automobile (1) au moyen d'un appareil médical (8) de l'occupant de véhicule (6),
dans lequel un signal comportant l'information médicale est transmis sans fil à un dispositif (4) agencé dans le véhicule automobile (1)
et dans lequel l'information médicale est transmise sans fil par le dispositif (4) à un dispositif (3) extérieur au véhicule automobile,
**caractérisé en ce que** le signal comportant l'information médicale est transmis sans fil et par voie acoustique, dans la plage des ondes sonores, au dispositif (4) agencé dans le véhicule automobile (1).

2. Procédé selon la revendication 1, **caractérisé en ce que** l'appareil médical (8) est relié de manière fixe, et donc pas en tant que capteur du véhicule automobile, au corps de l'occupant de véhicule (6) et/ou est conçu comme un appareil médical (8) implanté dans le corps de l'occupant de véhicule (6).

3. Procédé selon la revendication 1 ou 2, **caractérisé en ce que** le dispositif (3) extérieur au véhicule automobile est une base de données, en particulier d'un organisme de santé publique et/ou d'un fabricant de l'appareil médical (8).

4. Procédé selon l'une des revendications précédentes, **caractérisé en ce que** l'information médicale est évaluée au moyen du dispositif (3) extérieur au véhicule automobile et, en fonction d'un résultat d'évaluation transmis par le dispositif (3) extérieur au véhicule automobile au dispositif (4), une notification indiquant le résultat transmis est donnée à l'occupant de véhicule (6) et/ou à un autre occupant de véhicule (7) du véhicule automobile (1) dans le véhicule automobile (1), et une réaction de l'occupant de véhicule (6) et/ou de l'autre occupant de véhicule (7) à la première notification est déterminée en particulier par le dispositif (4).

5. Procédé selon la revendication 4, **caractérisé en ce que** la première notification est donnée de manière optique et/ou tactile et/ou acoustique.

6. Procédé selon l'une des revendications précédentes, **caractérisé en ce que** l'information médicale est évaluée au moyen du dispositif (3) extérieur au véhicule automobile et, en fonction d'un résultat de l'évaluation, une notification préparatoire concernant l'occupant de véhicule est donnée à un médecin dans un hôpital par le dispositif (3) extérieur au véhicule automobile.

7. Procédé selon l'une des revendications précédentes, **caractérisé en ce que** l'information médicale est évaluée au moyen du dispositif (3) extérieur au véhicule automobile et, en fonction d'un résultat de l'évaluation, une intervention dans la commande du véhicule automobile (1) est réalisée.

8. Procédé selon l'une des revendications précédentes, **caractérisé en ce que** le signal comportant l'information médicale est transmis sans fil et par voie acoustique dans la plage des ultrasons.

9. Système pour fournir une information médicale d'un occupant de véhicule (6) d'un véhicule automobile (1), dans lequel le système comprend un véhicule automobile (1) selon la revendication 11, et dans lequel le système est conçu de telle sorte que l'information médicale est transmise sans fil par le dispositif (4) au dispositif (3) extérieur au véhicule automobile.

10. Système selon la revendication 9, **caractérisé en ce que** le dispositif (3) extérieur au véhicule automobile est équipé pour évaluer l'information médicale et pour transmettre un résultat de l'évaluation au dispositif interne (4) du véhicule automobile, l'appareil médical (8) étant équipé pour adapter au moins un paramètre de l'appareil médical (8) en fonction du résultat transmis grâce à l'envoi d'un signal d'adaptation par le dispositif interne (4) à l'appareil médical (8).

11. Véhicule automobile (1) avec un dispositif interne (4), qui est conçu pour la communication sans fil d'un signal comportant une information médicale concernant l'occupant de véhicule au moyen d'un appareil médical (8) d'un occupant de véhicule (6) et pour la communication sans fil avec un dispositif (3) extérieur au véhicule automobile en vue de la transmission sans fil d'un signal comportant l'information médicale, lequel signal comportant l'information médicale est transmis sans fil et par voie acoustique, dans la plage des ondes sonores, au dispositif interne (4).
